(19) Europäisches Patentamt
European Patent Office
Office européen des brevets

(11) Publication number: **0 383 919**
**A1**

(12) **EUROPEAN PATENT APPLICATION**
**published in accordance with Art. 158(3) EPC**

(21) Application number: **89901739.6**

(22) Date of filing: **30.01.89**

(86) International application number:
**PCT/JP89/00086**

(87) International publication number:
**WO 89/07105 (10.08.89 89/18)**

(51) Int. Cl.5: **C07D 498/22**

(30) Priority: **04.02.88 JP 24571/88**

(43) Date of publication of application:
**29.08.90 Bulletin 90/35**

(84) Designated Contracting States:
**DE FR GB IT**

(71) Applicant: **KYOWA HAKKO KOGYO CO., LTD.**
**Ohtemachi Bldg., 6-1 Ohtemachi I-chome**
**Chiyoda-ku Tokyo 100(JP)**

(72) Inventor: **MURAKATA, Chikara**
**2-1-47-302, Negishidai**
**Asaka-shi Saitama 351(JP)**
Inventor: **SATO, Akira**
**1880-30, Kiso-machi**
**Machida-shi Tokyo 194(JP)**
Inventor: **KASAI, Masaji**
**3-12-15, Kugenumamatsugaoka**
**Fujisawa-shi Kanagawa 251(JP)**
Inventor: **MORIMOTO, Makoto**
**203-5, Shimotogari Nagaizumi-cho**
**Sunto-gun Shizuoka 411(JP)**
Inventor: **AKINAGA, Shiro**
**1188, Shimotogari Nagaizumi-cho**
**Sunto-gun Shizuoka 411(JP)**

(74) Representative: **Kinzebach, Werner, Dr. et al**
**Patentanwälte Reitstötter, Kinzebach und Partner Sternwartstrasse 4 Postfach 86 06 49**
**D-8000 München 86(DE)**

(54) **STAUROSPORIN DERIVATIVES.**

(57) Novel staurosporin derivatives represented by formula (I) (wherein $R^1$ represents hydrogen, lower alkyl, formyl or amino, $R^2$ represents hydrogen, formyl, lower alkanoyl or a group formed by removing a hydroxy moiety from the carboxyl group of an α-amino acid whose amino group may optionally be protected, one of $X^1$ and $X^2$ represents hydrogen and the other represents hydrogen, hydroxy, lower alkoxy or lower alkylthio, or $X^1$ and $X^2$ are taken together to form oxygen, provided that, when $R^1$ and $R^2$ are both hydrogen atoms and one of $X^1$ and $X^2$ is hydrogen, the other represents a group other than hydrogen or hydroxy) and their pharmacologically acceptable salts are disclosed. These compounds

EP 0 383 919 A1

inhibit protein kinase C and have a cell growth inhibiting activity.

SPECIFICATION

Staurosporine derivatives

Technical Field

This invention relates to a novel staurosporine derivative having protein kinase C-inhibitory and cell growth-inhibitory activities.

Background Technology

It is generally believed that protein kinase C is involved in cell growth and in the mechanism of carcinogenesis (e.g. Science, 225 1365; 1984). Therefore, it is expected that if protein kinase C activity can be artificially suppressed with a specific inhibitor, for instance, the prevention and treatment of tumors and the like will become feasible.

Meanwhile, staurosporine is an antibiotic having the following formula:

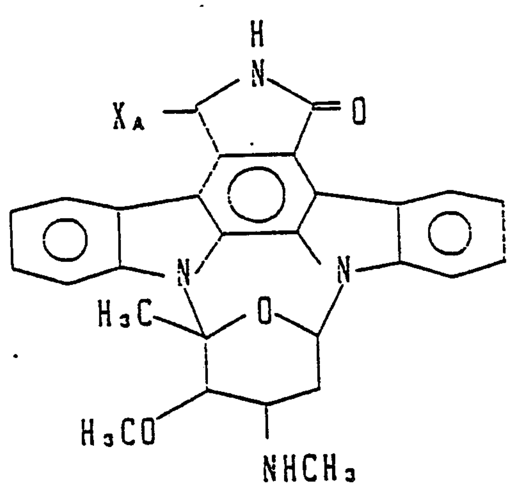

staurosporine: $X_A$ =H

UCN-01 : $X_A$ =OH

Staurosporine is not only known to show antibacterial activity against various bacteria but also known to show cell growth inhibitory activity against HeLa cells, human leukemia cells and so on (J. Antibiotics 30, 275, 1977; J. Chem. Soc. Chem. Commun., 800, 1978; JP-A-60-185719).

As a related compound of staurosporine, UCN-01 having antitumor activity is known (JP-A-62-220196).

The object of this invention is to provide a staurosporine derivative which inhibits protein kinase C and has excellent cell growth inhibitory activity.

Disclosure of the Invention

This invention relates to a staurosporine derivative of the following formula (I) (hereinafter referred to as compound (I); the same numbering applies to compounds of the other formulae)

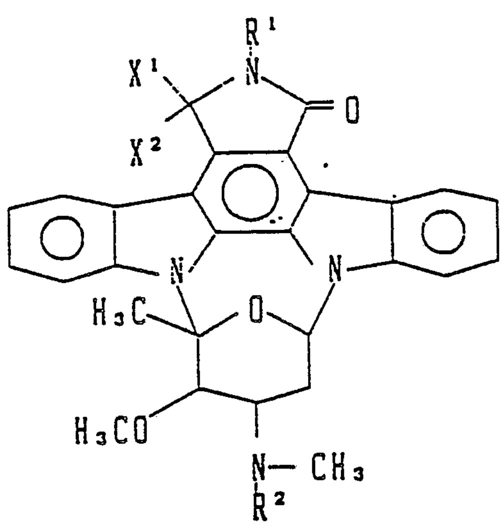

(I)

where $R^1$ represents hydrogen, lower alkyl, formyl or amino; $R^2$ represents hydrogen, formyl, lower alkanoyl or the group available upon elimination of the hydroxyl moiety of the carboxyl group of an α-amino acid, where the amino group of said amino acid may be protected by a protective group; one of $X^1$ and $X^2$ is hydrogen and the other is hydrogen, hydroxyl, lower alkoxyl or lower alkylthio or both of them, taken together, represent oxygen; provided, however, that where both $R^1$ and $R^2$ concurrently represent hydrogen and either one of $X^1$ and $X^2$ is hydrogen, the other is a group other than hydrogen and hydroxyl or a pharmaceutically acceptable salt thereof.

The lower alkyl and the lower alkyl moieties of lower alkoxyl and lower alkylthio in the definitions of groups in formula (I) include both straight-chain and branched alkyls containing 1 to 4 carbon atoms, such as methyl, ethyl, n-propyl, isopropyl, n-butyl, isobutyl, sec-butyl and tert-butyl. The lower alkanoyl in the definition of $R^2$ include both straight-chain and branched alkanoyls containing 2 to 5 carbon atoms, such as acetyl, propionyl, butyryl, isobutyryl and valeryl. The α-amino acid represented by $R^2$ includes glycine, alanine, proline and so on, and the protective group includes benzyloxycarbonyl, t-butoxycarbonyl and so on.

When compound (I) is a basic compound, it can be form acid addition salts. Examples of such acid addition salts of compound (I) include hydrochloride, hydrobromide, sulfate, nitrate, formate, acetate, benzoate, maleate, fumarate, succinate, tartarate, citrate, oxalate, methanesulfonate, p-toluenesulfonate, aspartate, glutamate and so on. While non-toxic pharmaceutically acceptable salts such as those acid addition salts mentioned above are preferred, other salts may also be of use for the isolation and purification of the product.

The compound of this invention is usually derived from an optically active staurosporine compound but all possible stereoisomers and mixtures thereof also fall within the scope of this invention.

A method for producing compound (I) is described below. It should be understood that the following is not an exclusive method for the production of compound (I).

It should also be understood that, in the production procedure described below, when any group defined hereinbefore undergoes change under the conditions of the procedure or is unsuitable for the procedure, the procedure can be facilitated by resort to techniques

which are commonly employed in organic synthetic chemistry, such as protection and deprotection of functional groups [e.g. Greene: Protective Groups in Organic Synthesis, John Wiley & Sons, Inc., 1981.]

By way of illustration, the starting compound staurosporine (IIa) can be reacted with benzyloxycarbonyl chloride (Cbz-Cl), a well-known amino-protecting group donor, to give compound (IIb).

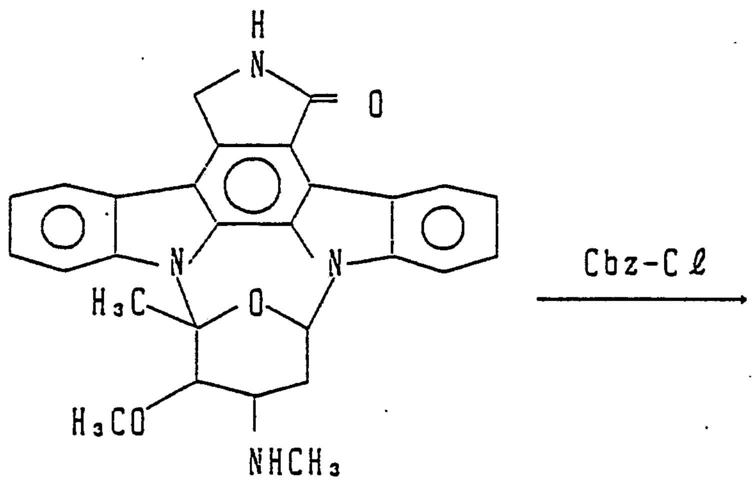


(II a)

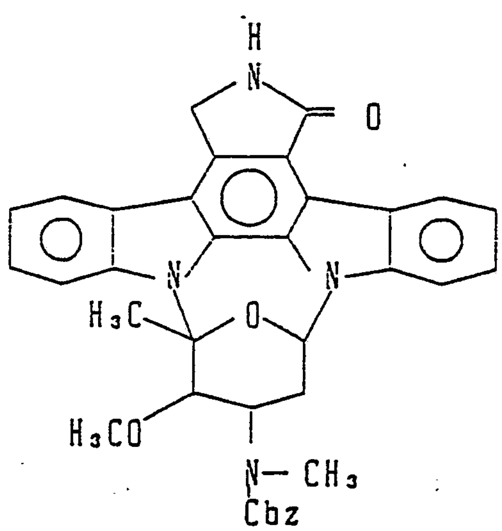

(II b)

Thus, compound (IIb) can be obtained by reacting compound (IIa) with Cbz-Cl in a solvent mixture of acetone and water in the presence of an appropriate base, such as sodium hydrogen carbonate, under ice-cooling for 0.5 to 1 hour.

Regarding compound (IIa), the base is used in a proportion of 1 to 5 equivalents and Cbz-Cl in a proportion of 1 to 1.5 equivalents.

Then, the objective compound can be obtained by subjecting compound (IIb) to either Process 1 or Process 3 described hereinafter and, then, to deprotection.

This deprotection can be accomplished by the conventional reduction reaction, such as catalytic reduction. Thus, in an inert solvent such as tetrahydrofuran (THF) or dimethylformamide (DMF), the catalytic reduction is carried out using a catalyst, which may generally be 5 to 10% palladium-on-carbon, in a hydrogen gas atmosphere at room temperature for one hour up to one day.

Process 1: Synthesis of $R^1$-modified compound (I-1)

1-1: Compound where $R^1$ is lower alkyl (I-1-1)

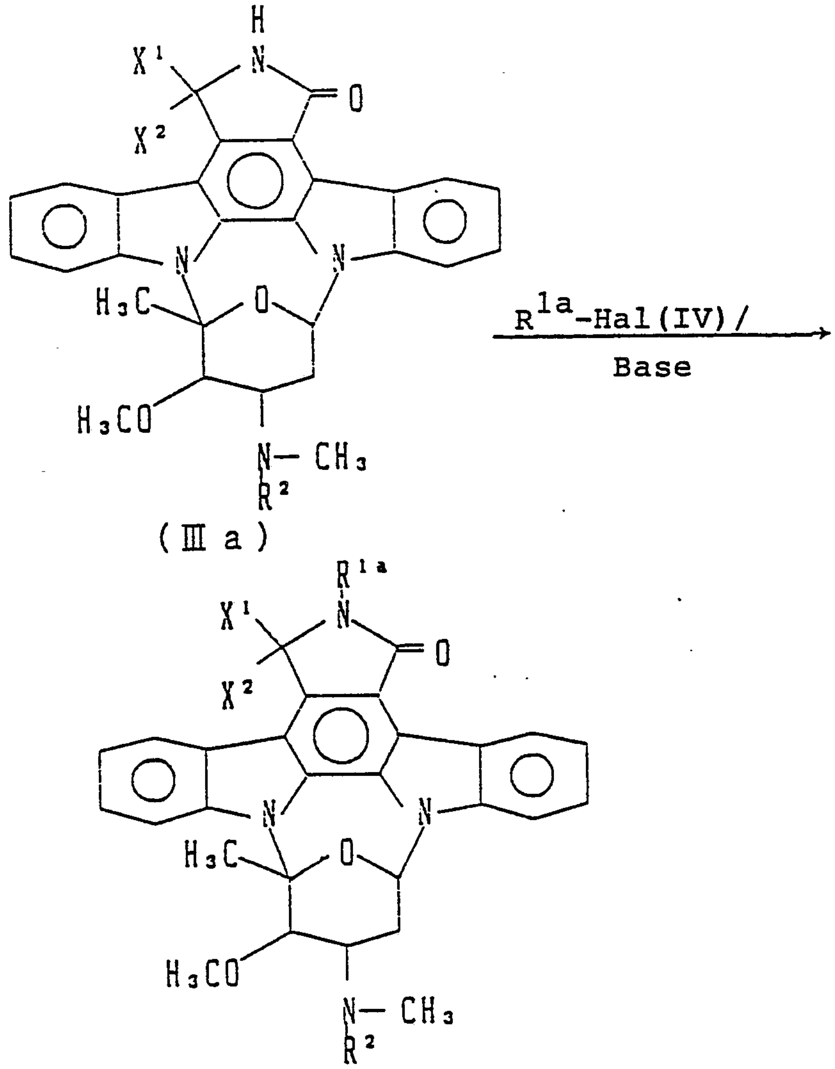


(III a)

(I - 1 - 1)

(where $R^2$, $X^1$ and $X^2$ are as defined above; $R^{1a}$ is lower alkyl in the definition of $R^1$; Hal is a halogen which is chlorine, bromine or iodine)

The compound (I-1-1) can be obtained by reacting compound (IIIa) [Compound (I) where $R^1$ is hydrogen, compound (IIa), compound (IIb), etc.] with 1 to 10 equivalents of an alkyl halide (IV) in a solvent, such

as DMF, in the presence of 1 to 1.5 equivalents of an appropriate base, such as sodium hydride, under ice-cooling. This reaction goes to completion generally in 15 minutes to 12 hours.

1-2: Compound wherein $R^1$ is formyl (I-1-2)

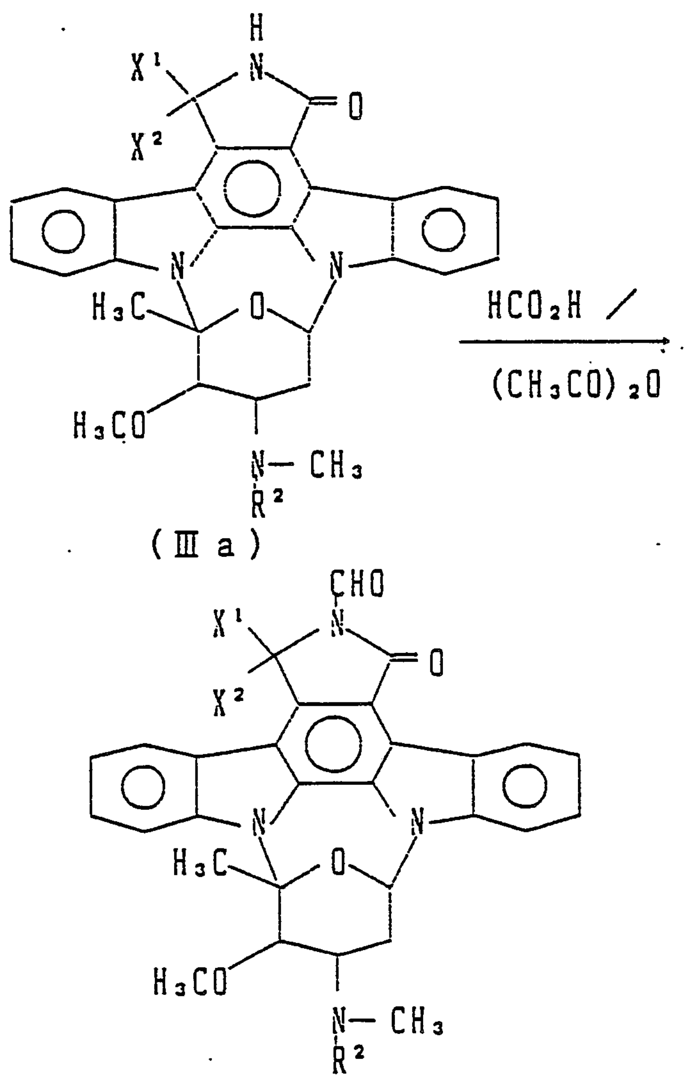


(III a)

(I - 1 - 2)

(where $R^2$, $X^1$ and $X^2$ are as defined above)

Compound (I-1-2) can be obtained by reacting

compound (IIIa) with a large excess or solvent amount of formic acid - acetic anhydride at room temperature for 12 to 24 hours.

1-3: Compound wherein $R^1$ is amino (I-1-3)

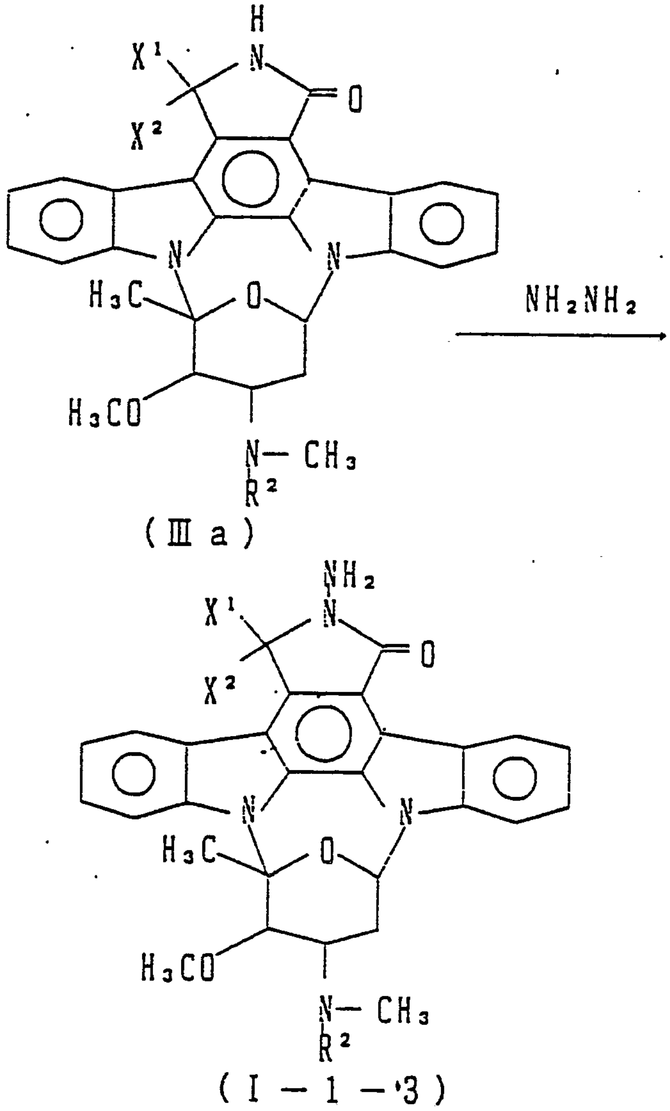


(III a)

(I - 1 - 3)

(where $R^2$, $X^1$ and $X^2$ are as defined above)

Compound (I-1-3) can be obtained by reacting compound (IIIa) with 10 to 50 equivalents of hydrazine

hydrate in an inert solvent, such as dioxane, at 70 to 110°C for 1 to 2 days.

Process 2: Synthesis of $R^2$-modified compound (I-2)

2-1: Compound wherein $R^2$ is formyl (I-2-1)

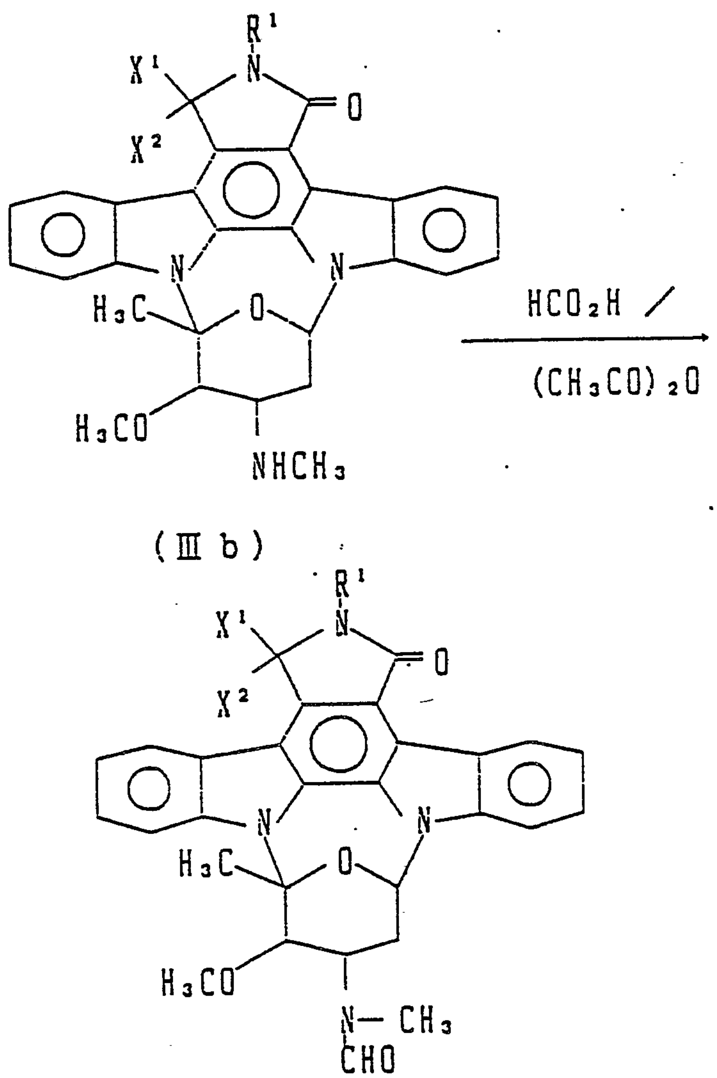


(III b)

(I - 2 - 1)

(where $R^1$, $X^1$ and $X^2$ are as defined above)

Compound (I-2-1) can be obtained by treating compound (IIIb) [compound (I) where $R^2$ is hydrogen, compound (IIa), etc.] in the same manner as Process 1-2.

2-2: Compound where $R^2$ is lower alkanoyl (I-2-2)

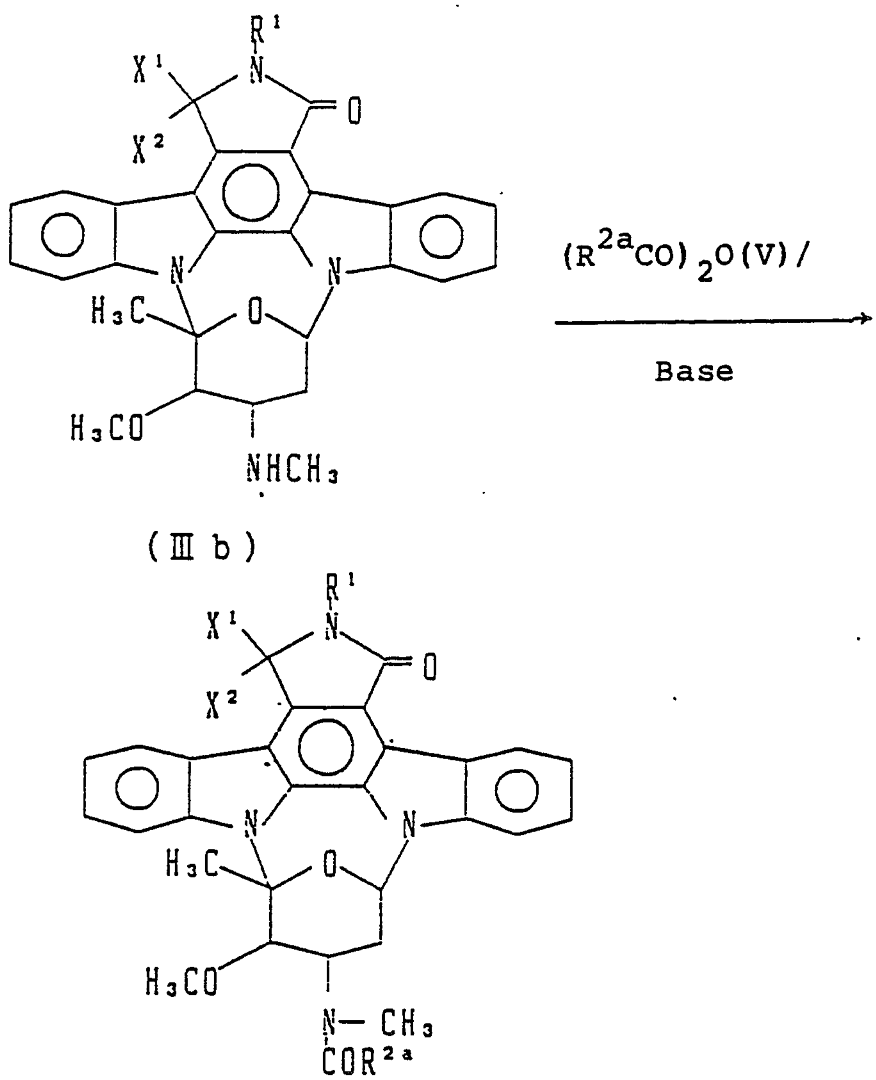


(III b)

(I - 2 - 2)

(where $R^1$, $X^1$ and $X^2$ are as defined above; $R^{2a}$ is lower alkyl)

The lower alkyl mentioned above has the same meaning as the alkyl defined before.

Compound (I-2-2) can be obtained by reacting compound (IIIb) with an acid anhydride (V) in an appropriate base, such as pyridine, at room temperature for 12 to 24 hours. Compound (V) is used in a proportion of 1 to 5 equivalents relative to compound (IIIb).

2-3: Compound wherein $R^2$ is an acyl group derived from an amino acid (I-2-3)

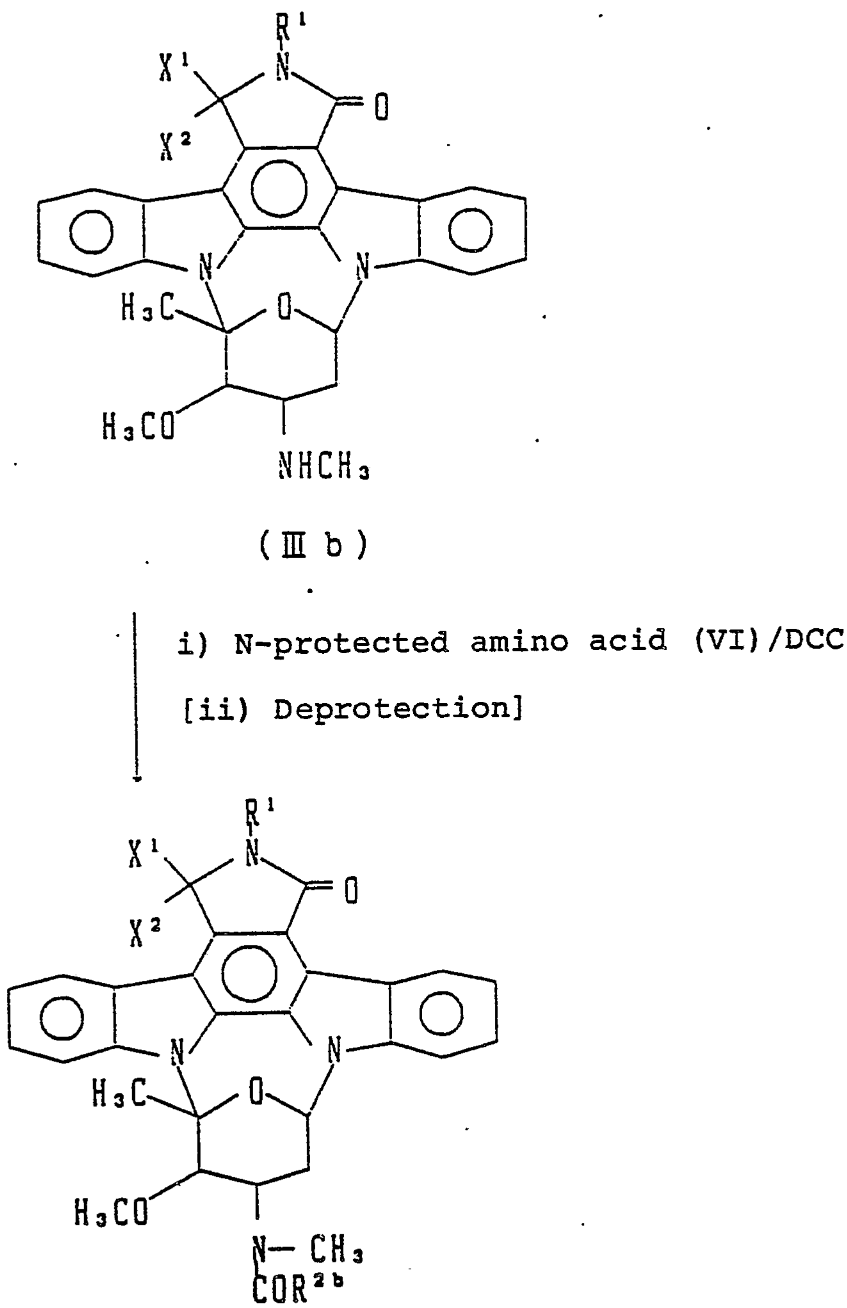


(III b)

(I-2-3)

(where $R^1$, $X^1$ and $X^2$ are as defined above; $R^{2b}$ is a group corresponding to the remainder of an α-amino acid after elimination of its carboxyl group, the amino group of which may be protected)

Compound (I-2-3) can be obtained by condensing compound (IIIb) with N-protected amino acid (VI) in THF using N-hydroxysuccinimide and dicyclohexylcarbodiimide (DCC). Based on compound (IIIb), compound (VI) is used in a proportion of 1 to 2 equivalents, N-hydroxysuccinimide in a proportion of 1 equivalent, and DCC in a proportion of 1 to 2 equivalents. This reaction is generally conducted at 0°C to room temperature and goes to completion in 1 hour up to one day.

When a compound having a free amino group (I-2-3a) is desired, the reaction product is deprotected by the conventional procedure. When the protective group is benzyloxycarbonyl, for instance, compound (I-2-3a) can be obtained by carrying out a catalytic reduction reaction in the same manner as described above.

Process 3: Synthesis of compound modified at $X^1$ and $X^2$ (I-3)

3-1: Compound where $X^1$ or $X^2$ is hydroxyl (I-3-1)

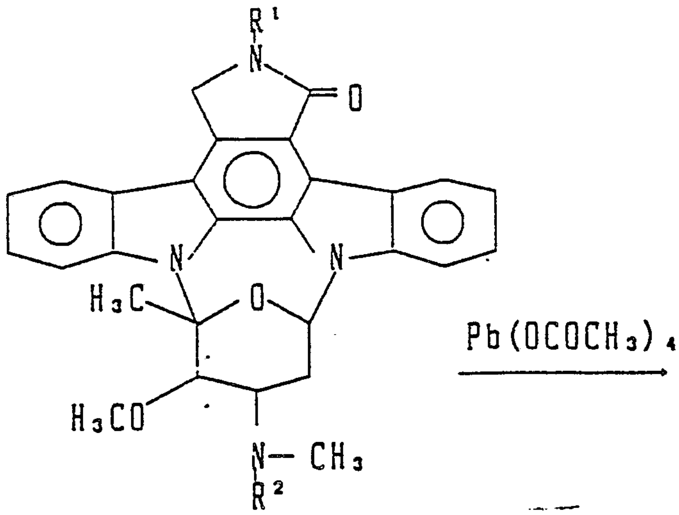


( I a )

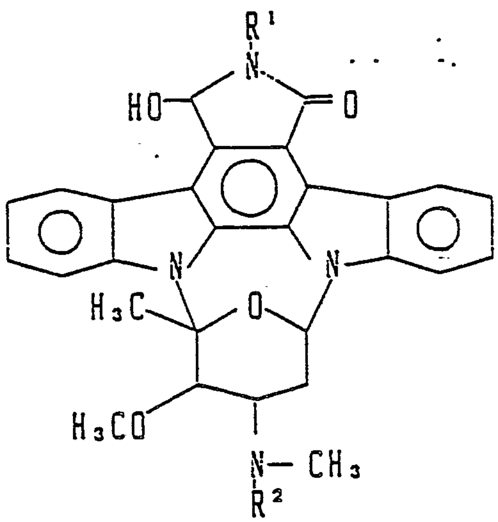

( I - 3 - 1 )

(where $R^1$ and $R^2$ are as defined above)

Compound (I-3-1) can be obtained by reacting compound (Ia) [compound (I) where $X^1$ and $X^2$ are hydrogen] with lead tetracetate in acetic acid at room temperature for 7 to 10 hours. Based on compound (Ia), lead tetracetate is used in a proportion of 1 to 2 equivalents.

3-2: Compound where $X^1$ or $X^2$ is lower alkoxyl or lower alkylthio (I-3-2)

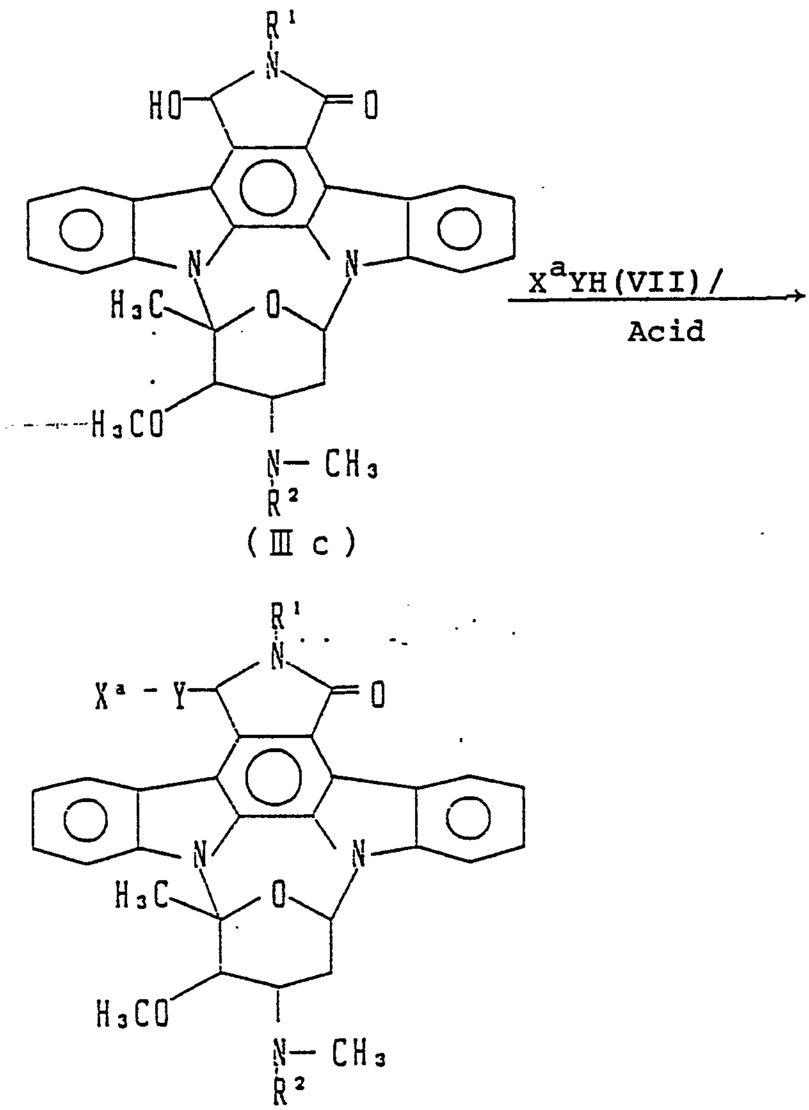


(III c)

(I - 3 - 2)

(where $R^1$ and $R^2$ are as defined above; $X^a$ is lower alkyl; Y is oxygen or sulfur)

The lower alkyl mentioned just above has the same meaning as defined before.

Compound (I-3-2) can be obtained by reacting

compound (IIIc) [compound (I-3-1) and UCN-01 and isomers thereof, for instance] with compound (VII) in an appropriate inert solvent, such as THF, in the presence of an appropriate acid catalyst, such as camphorsulfonic acid.

Based on compound (IIIc), compound (VII) is used in a proportion of at least 1 equivalent, preferably in a large excess, and the acid catalyst in a proportion of 0.02 to 0.05 equivalent. This reaction is generally conducted at room temperature and goes to completion in 0.5 to 1 hour.

3-3: Compound where $X^1$ and $X^2$, taken together, represent oxygen (I-3-3)

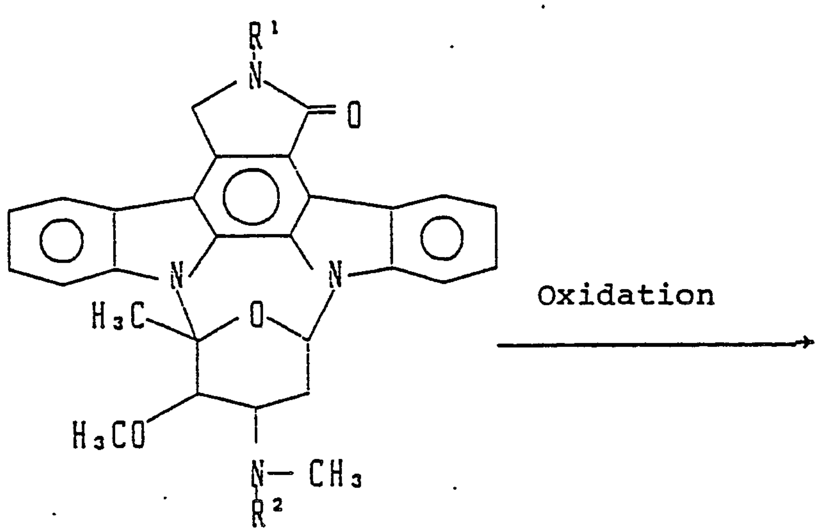


(IIId)

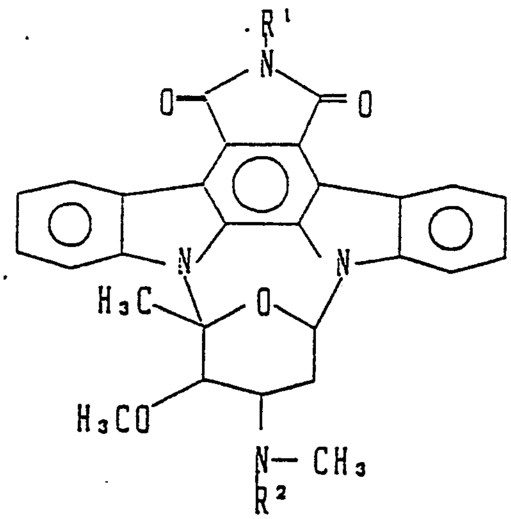

( I - 3 - 3 )

(where $R^1$ and $R^2$ are as defined above)

Compound (I-3-3) can be obtained by reacting compound (IIId) [compound (I) where $X^1$ and $X^2$ each is hydrogen, compound (IIa), compound (IIb), etc.] with an appropriate oxidizing agent, such as chromic acid, in pyridine at a temperature between 0°C and room temperature for 1 day. The oxidizing agent is used in a proportion of 5 to 7 equivalents per mole of compound (IIId).

By performing the above processes 1 to 3 in suitable combination, compound (I) having optional functional groups in optional positions can be obtained.

The isolation and purification of the reaction product after each of these processes can be accomplished by using the procedures commonly employed in organic synthesis, such as extraction, crystallization, various

kinds of chromatography, etc. in a suitable combination.

Compound (I) has marked cell growth inhibitory activity and, as such, provides antitumor preparations containing it as an active ingredient.

When compound (I) is used as an antitumor agent, respective species of compound (I) are dissolved in physiological saline or a glucose, lactose or mannitol solution for injection and administered generally as intravenous injections at the dose of 0.01 to 20 mg/kg. It is also possible to lyophilize them in accordance with the Pharmacopeia of Japan or formulate them with sodium chloride for use as powders for injection. Furthermore, these compounds may be formulated with the well-known pharmaceutically acceptable diluents, adjuvants and/or carriers, such as salts suited for pharmaceutical uses. For use as injections, there are cases in which auxiliary agents for enhancing the solubility of compound (I) are preferably incorporated. The dosage may be adjusted according to age and symptoms. The administration schedule can also be varied according to condition and dosage. For example, a once-a-day regimen (single dosing or repeated daily dosing) and an intermittent administration regimen of 1 to 3 times a week or once in 3 weeks may be mentioned. It is also possible to administer (I) intraarterially,

intraperitoneally or intrathoracically in the same dose range as mentioned above. Compound (I) may also be administered orally or rectally. In the latter cases, compound (I) may be formulated with appropriate adjuvants to provide tablets, powders, granules, syrups, suppositories and so on.

Representative examples of compound (I) obtained by the above-described processes are shown in Table 1 and the intermediates are shown in Table 2. Examples of production of these compounds (I) are given in the Examples, examples of production of the intermediates are given in Reference Examples, and examples of physiological activity tests with the representative examples of compound (I) are given in the Test Examples.

Table 1

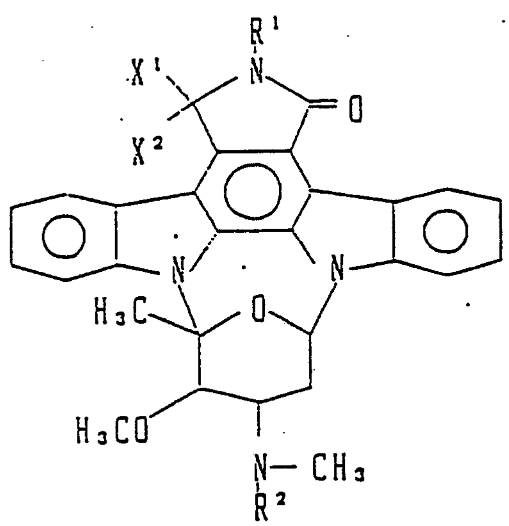

| Example No. | Compound No. | $R^1$ | $R^2$ | $X^1$ | $X^2$ | Salt |
|---|---|---|---|---|---|---|
| 1 | 1 | $CH_3$ | H | H | H | |
| 2 | 2 | CHO | CHO | H | H | |
| 2 | 3 | H | CHO | H | H | |
| 3 | 4 | CHO | H | H | H | |
| 4 | 5 | $NH_2$ | H | =O | | HCℓ |
| 5 | 6 | H | $COCH_3$ | H | H | |
| 6 | 7 | H | $COC_2H_5$ | H | H | |
| 7 | 8 | H | $CO\text{-}n\text{-}C_3H_7$ | H | H | |
| 8 | 9 | H | $COCH_2NHCbz$** | H | H | |
| 9 | 10 | H | $COCH_2NH_2$ | H | H | HCℓ |
| 10 | 11* | H | H | $OCH_3$ | H | |
| 11 | 12* | H | H | $SC_2H_5$ | H | |
| 12 | 13 | H | $COCH_3$ | =O | | |
| 13 | 14 | $CH_3$ | H | =O | | |
| 14 | 15 | H | H | =O | | |
| 15 | 16 | H | H | $OCH_3$ | H | |
| 15 | 17 | H | H | H | $OCH_3$ | |
| 16 | 18* | H | H | $O\text{-}i\text{-}C_3H_7$ | H | |
| 17 | 19* | H | H | $O\text{-}n\text{-}C_4H_9$ | H | |
| 18 | 20* | H | H | $OC_2H_5$ | H | |

* a mixture (approx. 1:1) of diastereomers

** Cbz stands for benzyloxycarbonyl (the same applies below).

Table 2 (intermediates)

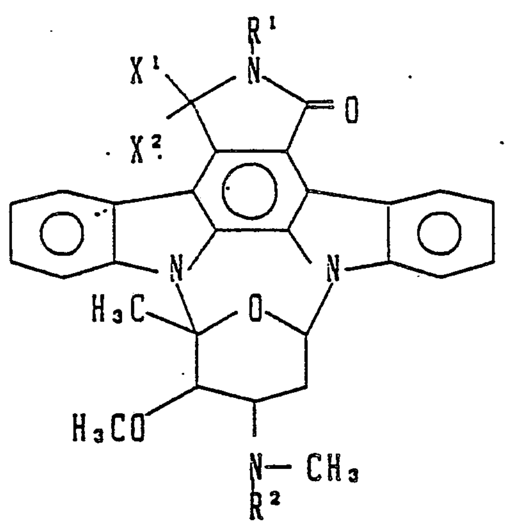

| Compound No. | Reference Example No. (Example No.) | $R^1$ | $R^2$ | $X^1$ | $X^2$ |
|---|---|---|---|---|---|
| a | 1 | H | Cbz | H | H |
| b | (1) | $CH_3$ | Cbz | H | H |
| c | (3) | CHO | Cbz | H | H |
| d | 2 | H | H | OH | H |
| e | (13) | $CH_3$ | Cbz | =O | |
| f | (14) | H | Cbz | =O | |

Example 1

In 18 ml of DMF was dissolved 360 mg (0.6 mmol) of compound a prepared in Reference Example 1, followed by the addition of 36 mg (0.9 mmol) of 60% sodium hydride. The mixture was stirred for 10 minutes, at the end of which time 0.054 ml (0.9 mmol) of methyl iodide was added and the mixture was stirred at room temperature for 0.5 hour. To this reaction mixture were added 5 ml of water and 30 ml of chloroform and the organic layer was separated, washed with saturated aqueous sodium chloride solution and dried over anhydrous magnesium sulfate. The solvent was distilled off under reduced pressure and the residue was purified by silica gel

column chromatography (chloroform). The procedure gave 312 mg (85%) of compound b.

Compound b:

$^1$H-NMR ($CDCl_3$) δ; 1.64 (s, 3H), 2.20-3.04 (m, 2H), 2.48 (s, 3H), 2.84 (s, 3H), 3.41 (s, 3H), 3.90-3.24 (m, 1H), 4.68-5.20 (m, 1H), 4.95 (s, 2H), 5.25 (s, 2H), 6.72 (t, 1H), 7.20-7.92 (m, 11H), 7.98 (d, 1H, J=8 Hz), 9.60 (d, 1H, J=8 Hz)

SI-MS (m/z); 615 $(M+1)^+$

In 3 ml of DMF was dissolved 70 mg (0.11 mmol) of the above compound b, and after addition of 60 mg of 10% palladium-on-carbon, the solution was stirred in a hydrogen stream at 40°C for 1 hour. The reaction mixture was filtered through Celite and the solvent was distilled off under reduced pressure. The residue was purified by silica gel column chromatography (2% methanol-chloroform) to give 27 mg (51%) of compound 1.

Compound 1:

$^1$H-NMR ($CDCl_3$) δ; 1.56 (s, 3H), 2.20-3.00 (m, 2H), 2.34 (s, 3H), 3.20-3.50 (m, 2H), 3.38 (s, 6H), 3.87 (d, 1H, J=4 Hz), 4.96 (s, 2H), 6.54 (m, 1H), 7.20-8.08 (m, 7H), 9.52 (d, 1H, J=8 Hz)

EI-MS (m/z); 480 $(M^+)$

Example 2

In a mixture of 3 ml formic acid and 1 ml acetic

anhydride was dissolved 249 mg (0.5 mmol) of staurosporine and the solution was stirred at room temperature overnight. To this reaction mixture were added 20 ml of water and 30 ml of chloroform, and the organic layer was separated, washed with saturated aqueous sodium hydrogen carbonate solution and saturated aqueous sodium chloride solution and dried over anhydrous magnesium sulfate. The solvent was then distilled off under reduced pressure and the residue was purified by silica gel column chromatography (chloroform) to give 25 mg (9%) of compound 2 and 33 mg (12.5%) of compound 3.

Compound 2:

$^1$H-NMR ($CDCl_3$) δ; 2.30 (s, 3H), 2.54 (s, 3H), 2.84 (s, 3H), 2.20-2.86 (m, 2H), 3.96 (br.s, 1H), 4.72-5.36 (m, 3H), 6.68 (m, 1H), 7.12-8.04 (m, 7H), 8.14 (s, 1H), 9.25 (d, 1H, J=8 Hz), 9.37 (s, 1H),

SI-MS (m/z); 523 $(M+1)^+$

Compound 3:

$^1$H-NMR ($CDCl_3$) δ; 2.28-2.88 (m, 2H), 2.36 (s, 3H), 2.48 (s, 3H), 2.82 (s, 3H), 3.95 (d, 1H, J=2 Hz), 4.76-5.16 (m, 1H), 4.94 (s, 2H), 6.54 (br.s, 1H), 6.65 (br.t, 1H), 7.04-7.60 (m, 5H), 7.72 (d, 1H, J=8 Hz), 7.88 (d, 1H, J=8 Hz), 8.11

(s, 1H), 9.44 (d, 1H, J=8 Hz)

SI-MS (m/z); 495 $(M+1)^+$

Example 3

By substantially the same procedure as Example 2, 50 mg (40%) of compound c was obtained from 120 mg (0.2 mmol) of compound a.

By the same catalytic reduction procedure as described in Example 1 using 50 mg of the above compound c without purification, 30 mg (77%) of compound 4 was obtained.

Compound 4:

$^1$H-NMR ($CDCl_3$+DMSO-$d_6$) δ; 1.60 (s, 3H), 2.20-3.00 (m, 2H), 2.40 (s, 3H), 3.20-3.64 (m, 1H), 3.30 (s, 3H), 3.92 (d, 1H, J=4 Hz), 5.04 (s, 2H), 6.48 (m, 1H), 7.12-8.08 (m, 7H), 9.10 (d, 1H, J=8 Hz), 9.25 (s, 1H)

SI-MS (m/z); 495 $(M+1)^+$

Example 4

In 15 ml of dioxane was dissolved 240 mg (0.5 mmol) of compound 15 obtained in Example 14, followed by addition of 1.21 ml of hydrazine hydrate, and the mixture was heated at 100°C for 2 days. The solvent was then distilled off and the residue was purified by silica gel chromatography (chloroform-methanol-28% aqueous ammonia = 97:3:0.1). The eluate was treated

with 1.7N hydrochloric acid-ethyl acetate to give 207 mg (84%) of hydrochloride compound 5.

Compound 5:

$^1$H-NMR (DMSO-$d_6$) δ; 1.92-2.76 (m, 2H), 2.24 (s, 3H), 2.47 (s, 3H), 2.67 (br.s, 3H), 3.84-4.50 (m, 4H), 4.60 (br.s, 2H), 6.94 (m, 1H), 7.28-7.72 (m, 5H), 8.08 (d, 1H, J=8 Hz), 9.09 (d, 1H, J=8 Hz), 9.30 (d, 1H, J=8 Hz)

SI-MS (m/z); 496 $(M+1)^+$

Example 5

In 3 ml of pyridine was dissolved 50 mg of staurosporine followed by addition of 0.1 ml of acetic anhydride, and the mixture was stirred at room temperature for 3.5 hours. The solvent was distilled off under reduced pressure and the residue was diluted with 10 ml of chloroform, washed with 5% hydrochloric acid and saturated aqueous sodium chloride solution, and dried over anhydrous magnesium sulfate. Finally, the solvent was distilled off under reduced pressure to give 27 mg (50%) of compound 6.

Compound 6:

$^1$H-NMR ($CDCl_3$) δ; 2.00-2.96 (m, 2H), 2.14 (s, 3H), 2.44 (s, 3H), 2.47 (s, 3H), 2.83 (s, 3H), 4.01 (d, 1H, J=3 Hz), 4.99 (s, 2H), 5.23 (m, 1H), 6.70 (t, 1H), 7.16-8.00 (m, 7H), 8.68 (m,

1H), 9.50 (dd, 1H, J=2, 8 Hz)

SI-MS (m/z); 509 $(M+1)^+$

Example 6

Using 249 mg (0.5 mmol) of staurosporine and propionic anhydride, the procedure of Example 5 was repeated to give 80 mg (29%) of compound 7.

Compound 7:

$^1$H-NMR ($CDCl_3$) δ; 1.16 (t, 3H, J=8 Hz), 2.04-2.92 (m, 4H), 2.44 (s, 3H), 2.48 (s, 3H), 4.00 (br.s, 1H), 4.97 (s, 2H), 5.20 (m, 1H), 6.68 (m, 2H), 7.16-7.60 (m, 5H) 7.74 (d, 1H, J=8 Hz), 7.90 (d, 1H, J=8 Hz), 9.44 (d, 1H, J=8 Hz)

SI-MS (m/z); 523 $(M+1)^+$

Example 7

Using 249 mg (0.5 mmol) of staurosporine and butyric anhydride, the procedure of Example 5 was repeated to give 60 mg (21%) of compound 8.

Compound 8:

$^1$H-NMR ($CDCl_3$) δ; 0.98 (t, 3H, J=8 Hz), 1.66 (m, 2H), 2.20-3.00 (m, 4H), 2.46 (s, 3H), 2.50 (s, 3H), 2.84 (s, 3H), 4.03 (br.s, 1H), 5.01 (s, 2H), 4.96-5.36 (m, 1H), 6.44 (br.s, 1H), 6.72 (t, 1H, J=8 Hz), 7.20-7.60 (m, 5H), 7.75 (dd, 1H, J=2, 8 Hz), 7.93 (d, 1H, J=8 Hz), 9.45 (d, 1H, J=7 Hz)

SI-MS (m/z); 537 $(M+1)^+$

Example 8

In 25 ml of THF was dissolved 466 mg (1 mmol) of staurosporine followed by addition of 522 mg (2.5 mmol) of N-Cbz-glycine, 0.32 ml (3 mmol) of N-methylmorpholine and 32.7 mg (2.5 mmol) of N-hydroxysuccinimide. Then, under ice-cooling, a solution of 515 mg (2.5 mmol) of DCC in 10 ml of THF was added. The mixture was stirred at room temperature overnight. The reaction mixture was then filtered through Celite and the solvent was distilled off. Finally, the residue was purified by silica gel column chromatography (2% methanol-chloroform) to give 690 mg (100%) of compound 9.

Compound 9:

$^1$H-NMR ($CDCl_3$-DMSO-$d_6$) δ; 2.20-3.20 (m, 2H), 2.44 (s, 3H), 2.84 (s, 3H), 3.04 (s, 3H), 3.95 (d, 1H, J=6 Hz), 4.05 (br.s, 1H), 4.24-4.84 (m, 1H), 4.96 (s, 2H), 5.10 (s, 2H), 6.52 (m, 1H), 6.80 (t, 3H, J=8 Hz), 7.16-8.00 (m, 12H), 9.37 (d, 1H, J=8 Hz)

SI-MS (m/z); 656 $(M-1)^+$, 657 $(M^+)$, 658 $(M+1)^+$

Example 9

Using 660 mg of compound 9 obtained in Example 8, the catalytic reduction reaction was carried out as in Example 1 to give 120 mg (21%) of compound 10.

Compound 10:

$^1$H-NMR (DMSO-$d_6$) δ; 2.16-2.90 (m, 2H), 2.36 (s, 3H), 2.68 (s, 3H), 2.78 (s, 3H), 3.00-3.68 (m, 1H), 3.90 (m, 2H), 4.16 (br.s, 1H), 5.00 (br.s, 2H), 6.84-8.60 (m, 11H), 9.26 (d, 1H, J=8 Hz)

SI-MS (m/z); 524 $(M+1)^+$

Example 10

In 3 ml of THF was dissolved 30 mg (0.06 mmol) of compound d obtained in Reference Example 2, followed by addition of 1 ml of methanol and 18.6 mg (0.08 mmol) of camphorsulfonic acid, and the mixture was refluxed for 1 hour. The reaction mixture was diluted with 20 ml of ethyl acetate, washed successively with saturated aqueous sodium hydrogen carbonate solution and saturated aqueous sodium chloride solution, and dried over anhydrous magnesium sulfate. The solvent was then distilled off under reduced pressure and the residue was purified by high performance liquid chromatography. The procedure gave 24 mg (81%) of compound 11 as a mixture of diastereomers.

Compound 11:

$^1$H-NMR ($CDCl_3$) δ; 1.54 and 1.57 (s, 3H), 2.27 and 2.36 (s, 3H), 2.38-2.45 (m, 2H), 2.68-2.77 (m, 1H), 3.07 and 3.14 (s, 3H), 3.55 (m, 1H),

3.38 and 3.41 (s, 3H), 3.87 (m, 1H), 6.21 and 6.23 (s, 1H), 6.54 and 6.55 (s, 3H), 6.61 (dd, 1H, J=1.5, 8.2 Hz), 7.28-7.51 (m, 5H), 7.91 (dd, 1H, J=2.8, 8.5 Hz), 8.40-8.44 (m, 1H), 9.36 (d, 1H, J=8 Hz)

SI-MS (m/z); 497 $(M+1)^+$

Example 11

The procedure of Example 10 was repeated using compound d of Reference Example 2 and ethyl mercaptan, in lieu of methanol, to give 22 mg (70%) of compound 12 as a mixture of diastereomers.

Compound 12:

$^1$H-NMR ($CDCl_3$) δ; 0.77 and 0.99 (t, 3H, J=7.5 Hz), 1.54 and 1.56 (s, 3H), 2.27 and 2.36 (s, 3H), 2.30-2.44 (m, 4H), 2.64-2.76 (m, 1H), 3.33 (m, 1H), 3.36 and 3.37 (s, 3H), 3.87 (dd, 1H, J=3.8 Hz), 6.23 and 6.26 (s, 1H), 6.40 and 6.46 (s, 1H), 6.53 (d, 1H, J=5.8 Hz), 7.25-7.51 (m, 5H), 7.92 (t, 1H, J=7.9 Hz), 8.42 (m, 1H), 9.41 (d, 1H, J=8.0 Hz)

SI-MS (m/z); 527 $(M+1)^+$

Example 12

To 3 ml of pyridine was added 385 mg (3.85 mmol) of chromic acid with ice-cooling, followed by addition of 5 ml of a pyridine solution of 300 mg (0.59 mmol) of

compound 6 of Example 5, and the mixture was stirred at room temperature overnight. The reaction mixture was filtered through Celite and the solvent was distilled off under reduced pressure. Finally the residue was purified by silica gel column chromatography (1% methanol-chloroform) to give 208 mg (68%) of compound 13.

Compound 13:

$^1$H-NMR ($CDCl_3$) δ; 2.00-3.04 (m, 2H), 2.15 (s, 3H), 2.32 (s, 3H), 2.45 (s, 3H), 2.84 (s, 3H), 3.94 (br.s, 1H), 5.24 (m, 1H), 6.68 (m, 1H), 7.16-7.80 (m, 6H), 8.00 (br.s, 1H), 9.24 (d, 1H, J=8 Hz), 9.40 (d, 1H, J=8 Hz)

EI-MS (m/z); 522 ($M^+$)

Example 13

Using 232 mg (0.38 mmol) of compound b of Example 1, the procedure of Example 12 was repeated to give 160 mg (67%) of compound e.

Compound e:

$^1$H-NMR ($CDCl_3$) δ; 1.53 (s, 3H), 2.20-2.92 (m, 2H), 2.36 (s, 3H), 2.79 (s, 3H), 3.25 (s, 3H), 3.90 (m, 1H), 4.60-5.00 (m, 1H), 5.20 (s, 2H), 6.65 (dd, 1H, J=7, 8 Hz), 7.16-7.80 (m, 11H), 9.28 (d, 1H, J=8 Hz), 9.44 (d, 1H, J=8 Hz)

SI-MS (m/z); 629

Using 140 mg of the above compound e, the catalytic reduction reaction of Example 1 was repeated to give 96 mg (89%) of compound 14.

Compound 14:

$^1H$-NMR ($CDCl_3$-DMSO-$d_6$) δ; 2.00-4.12 (m, 5H), 2.24 (s, 3H), 2.46 (s, 3H), 2.70 (br.s, 3H), 3.14 (s, 3H), 4.66 (br.s, 1H), 6.74 (m, 1H), 7.08-8.04 (m, 6H), 9.14 (d, 1H, J=8 Hz), 9.34 (d, 1H, J=8 Hz)

EI-MS (m/z); 494 ($M^+$)

Example 14

Using 70 mg (0.1 mmol) of compound a of Reference Example 1, the procedure of Example 12 was repeated to give 26 mg (43%) of compound f. Then, using 20 mg of this compound f, the catalytic reduction process of Example 1 was repeated to give 4 mg (27%) of compound 15.

Compound 15:

$^1H$-NMR ($CDCl_3$) δ; 1.60 (s, 3H), 2.12-2.84 (m, 2H), 2.36 (s, 3H), 3.20-3.60 (m, 2H), 3.36 (s, 3H), 3.92 (d, 1H, J=4 Hz), 6.56 (m, 1H), 7.24-8.04 (m, 7H), 9.32 (d, 1H, J=8 Hz), 9.44 (d, 1H, J=8 Hz)

EI-MS (m/z); 480 ($M^+$)

Example 15

In 16 ml of THF was dissolved 200 mg of the mixture of compound d and UCN-01 obtained in Reference Example 2, followed by addition of 4 ml of methanol and 125 mg of camphorsulfonic acid, and the mixture was refluxed for 1 hour. The reaction mixture was diluted with ethyl acetate, washed successively with saturated aqueous sodium hydrogen carbonate solution and saturated aqueous sodium chloride solution, and dried over anhydrous magnesium sulfate. The solvent was then distilled off under reduced pressure and the residue was purified by silica gel column chromatography (chloroform). The procedure gave 55 mg (27%) of compound 16, 39 mg (19%) of compound 17, and 53 mg (26%) of a mixture of compounds 16 and 17.

Compound 16:

$^1$H-NMR ($CDCl_3$) δ; 1.55 (s, 3H), 2.37 (s, 3H), 2.39 (ddd, 1H, J=3.7, 5.7, 14.7 Hz), 2.74 (ddd, 1H, J=1.3, 4.1, 14.7 Hz), 3.14 (s, 3H), 3.35 (m, 1H), 3.41 (s, 3H), 3.89 (d, 1H, J=3.5 Hz), 6.28 (br.s, 1H), 6.55 (dd, 1H, J=1.2, 5.7 Hz), 6.60 (d, 1H, J=1.6 Hz), 7.29 (m, 1H), 7.35 (m, 1H), 7.37 (m, 1H), 7.43 (m, 1H), 7.50 (m, 1H), 7.92 (d, 1H, J=8.5 Hz), 8.42 (ddd, 1H, J=0.5, 1.3, 7.9 Hz), 9.36 (ddd, 1H, J=0.7, 1.2, 7.9 Hz)

SI-MS (m/z); 497 $(M+1)^+$, 465, 368, 336, 309

Compound 17:

$^1$H-NMR ($CDCl_3$) δ; 1.58 (s, 3H), 2.37 (s, 3H), 2.42 (ddd, 1H, J=3.8, 5.9, 14.7 Hz), 2.71 (ddd, 1H, J=1.3, 4.3, 14.7 Hz), 3.08 (s, 3H), 3.34 (m, 1H), 3.38 (s, 3H), 3.87 (d, 1H, J=3.5 Hz), 6.26 (br.s, 1H), 6.55 (dd, 1H, J=1.2, 5.8 Hz), 6.62 (d, 1H, J=1.6 Hz), 7.29 (d, 1H, J=8.3 Hz), 7.33 (m, 1H), 7.37 (m, 1H), 7.43 (m, 1H), 7.50 (m, 1H), 7.91 (d, 1H, J=8.5 Hz), 8.44 (ddd, 1H, J=0.5, 0.8, 7.9 Hz), 9.36 (ddd, 1H, J=0.7, 1.1, 7.9 Hz)

SI-MS (m/z); 497 $(M+1)^+$, 465, 409, 368, 336, 309

Example 16

The procedure of Example 15 was repeated except that 100 mg of the mixture of compound d and UCN-01 obtained in Reference Example 2 and isopropyl alcohol, which was in lieu of methanol, were used to give 56 mg (49%) of compound 18 as a mixture of diastereomers.

Compound 18:

$^1$H-NMR ($CDCl_3$) δ; 0.77 and 0.91 (d, 3H, J=6.2 Hz), 1.26 and 1.27 (d, 3H, J=6.2 Hz), 1.72 and 1.80 (br. 3H), 2.38 (s, 3H), 2.52-2.57 (m, 1H), 2.64 and 2.72 (m, 1H), 3.20-3.23 (br. 3H), 3.41 (br. 1H), 3.85 (m, 1H), 3.97 (m, 1H), 6.46 and

6.52 (br. 1H), 6.56-6.59 (m, 2H), 7.27-7.49 (m, 5H), 7.86 and 7.88 (d, 1H, J=8.3 Hz), 8.39 and 8.41 (dd, 1H, J=0.8, 7.9 Hz), 9.32 and 9.34 (d, 1H, J=8 Hz)

EI-MS (m/z); 524 ($M^+$), 466, 395, 336, 156

Example 17

The procedure of Example 15 was repeated except that 100 mg of the mixture of compound d and UCN-01 obtained in Reference Example 2 and n-butanol, which was in lieu of methanol, were used to give 66 mg (57%) of compound 19 as a mixture of diastereomers.

Compound 19:

$^1$H-NMR ($CDCl_3$) δ; 0.75 and 0.79 (t, 3H, J=7.3 Hz), 1.22-1.32 (m, 2H), 1.41-1.53 (m, 2H), 1.56 and 1.64 (s, 3H), 2.36 and 2.37 (s, 3H), 2.41-2.48 (m, 1H), 2.68-2.77 (m, 1H), 3.03 and 3.14 (m, 1H), 3.36 and 3.39 (s, 3H), 3.52 (m, 1H), 3.89 (m, 1H), 6.27 and 6.28 (br.s, 1H), 6.55 (m, 1H), 6.58 and 6.60 (d, 1H, J=1.5 Hz), 7.28-7.51 (m, 5H), 7.90 and 7.91 (d, 1H, J=8.5 Hz), 8.42-8.47 (m, 1H), 9.36 (dd, 1H, J=0.7, 7.9 Hz)

EI-MS (m/z); 538 ($M^+$), 466, 409, 336, 156

Example 18

The procedure of Example 15 was repeated except

that 50 mg of the mixture of compound d and UCN-01 obtained in Reference Example 2 and ethanol, in lieu of methanol, were used to give 37 mg (73%) of compound 20 as a mixture of diastereomers.

Compound 20:

$^1$H-NMR ($CDCl_3$) δ; 1.11 and 1.13 (t, 3H, J=7.1 Hz), 1.56 and 1.61 (s, 3H), 2.36 and 2.37 (s, 3H), 2.42 (m, 1H), 2.69-2.77 (m, 1H), 3.12 and 3.23 (m, 1H), 3.36 (m, 1H), 3.38 and 3.40 (s, 3H), 3.60 (m, 1H), 3.89 (m, 1H), 6.27 and 6.28 (br.s, 1H), 6.55 (m, 1H), 6.58 and 6.60 (d, 1H, J=1.5 Hz), 7.28-7.51 (m, 5H), 7.91 (m, 1H), 8.46 (m, 1H), 9.35 (d, 1H, J=8.0 Hz)

EI-MS (m/z); 510 ($M^+$), 465, 381, 336, 185, 156

Reference example 1

In a mixture of 30 ml acetone and 20 ml water was dissolved 932 mg (2 mmol) of staurosporine followed by addition of 840 mg (10 mmol) of sodium hydrogen carbonate. Then, 0.43 ml (3 mmol) of benzyloxycarbonyl chloride was added with ice-cooling and the mixture was stirred at the same temperature for 0.5 hour. The reaction mixture was diluted with 50 ml of ethyl acetate, washed with water and saturated aqueous sodium chloride solution, and dried over anhydrous magnesium sulfate. Finally the solvent was distilled off under

reduced pressure to give 1.17 g (98%) of compound a.

Compound a:

$^1$H-NMR ($CDCl_3$) δ; 1.63 (s, 3H), 2.07-2.93 (m, 2H), 2.46 (s, 3H), 2.79 (s, 3H), 3.99 (m, 1H), 4.60-5.10 (m, 1H), 4.97 (s, 2H), 5.17 (s, 2H), 6.46 (s, 1H), 6.66 (t, 1H), 7.10-8.00 (m, 12H), 9.43 (d, 1H, J=8 Hz)

SI-MS (m/z); 601 $(M+1)^+$

Reference Example 2

In 115 ml of acetic acid was dissolved 5.41 g (9.0 mmol) of compound a of Reference Example 1 followed by addition of 6.0 g (13.5 mmol) of lead tetraacetate, and the mixture was stirred at room temperature in the dark for 6 hours. To the reaction mixture was added 150 ml of water and the resulting precipitate was recovered by filtration. The precipitate was crudely purified by silica gel column chromatography (chloroform-methanol-28% aqueous ammonia = 99:1:0.1) to give 1.68 g (40%) of the hydroxy compound (I-3-1; $R^1$=H, $R^2$=Cbz).

The above compound was subjected to the catalytic reduction described in Example 1 and the reaction product was purified by silica gel column chromatography (chloroform-methanol-28% aqueous ammonia = 99:1:0.1) to give 88 mg (9%) of UCN-01 and 221 mg (23%) of compound d.

UCN-01:

SI-MS (m/z); 483 $(M+1)^+$, 465

Compound d:

SI-MS (m/z); 483 $(M+1)^+$, 465

Incidentally, UCN-01 and compound d are diastereomers such that the orientations of $X^1$ and $X^2$ are anti to each other.

Test Example 1

The cell growth inhibitory activity of compound (I) according to this invention was tested by the following methods. The results are shown in Table 3.

(1) HeLa $S_3$ cell growth inhibition assay:

A suspension of HeLa $S_3$ cells ($3 \times 10^4$ cells/ml) in MEM containing 10% fetal serum and 2 mM glutamine is distributed in 0.1 ml portions into the wells of a 96-well microtiter plate and incubated overnight in a $CO_2$ incubator at 37°C. Then, 0.05 ml portions of an appropriately diluted solution of the test compound are added. The cells are further cultured in the $CO_2$ incubator and the culture supernatant is discarded. After washing with PBS (-) once, 0.1 ml portions of fresh medium are added to the wells and the incubation is continued in the $CO_2$ incubator at 37°C for 72 hours. After the culture supernatant is discarded, 0.1 ml portions of culture medium containing 0.02% neutral

red are added to the respective wells and the microtiter plate is incubated in the $CO_2$ incubator at 37°C for 1 hour to stain the cells. After removal of the culture supernatant, the plate is washed with physiological saline once and the dye is extracted with 0.001 N HCl/30% ethanol. Then, using a microplate reader, the absorbances at 550 nm are measured. By comparing the absorbance of intact cells with the absorbances of cells treated with known concentrations of the test compound, the 50% growth inhibition concentration of the compound is calculated and designated as $IC_{50}$.

(2) CoLo 320DM cell growth inhibition assay:

A suspension of CoLo 320DM cells ($10^5$ cells/ml) in RPMI 1640 medium containing 10% fetal bovine serum, 100 U/ml penicillin and 100 μg/ml streptomycin is distributed in 0.1 ml portions into the wells of a 96-well microtiter plate. Then, the same procedure as (1) is followed and the counting of cells is carried out with a microcell counter. By comparing the number of intact cells with that of cells treated with known concentrations of the test compound, a 50% cell growth inhibition concentration of the compound is calculated and designated as $IC_{50}$.

Table 3

| Compound No. | $IC_{50}$ (μg/ml) | |
|---|---|---|
| | HeLa $S_3$ | CoLo 320DM |
| 4 | 0.18 | 0.19 |
| 5 | 0.75 | 0.59 |
| 10 | 0.051 | 0.55 |
| 11 | 0.17 | 0.14 |
| 12 | 0.16 | 0.088 |
| 14 | 0.78 | > 1 |
| 16 | 0.20 | -- |
| 17 | 0.16 | -- |
| 18 | 0.29 | -- |
| 19 | 0.32 | -- |
| 20 | 0.29 | -- |
| Staurosporine (Reference compound) | 0.05 | 0.018 |
| UCN-01 (Reference compound) | 0.17 | -- |

Test Example 2

(1) Protein kinase C inhibition assay:

According to the method of Kikkawa et al. (J. Biol. Chem. 257, 13341, 1982), 10 μl of a solution of the test compound is added to 250 μl of a solution containing 2.5 μmole magnesium acetate, 50 μg of histone

type IIS (Sigma), 20 µg of phosphatidylserine, 0.8 µg of diolein, 25 nmole calcium chloride, 5 µg of a crude enzyme (partially purified from the rat brain by the method of Yoshikawa et al.) and 5 µmole Tris-HCl buffer (pH 7.5) and the mixture is incubated at 30°C for 3 minutes. Then, 1.25 nmole [$\gamma$-$^{32}$P]ATP (5-10 x $10^3$ cpm/nmole) is added and the phosphorylation reaction is carried out at 30°C for 30 minutes. The reaction is terminated by addition of 25% trichloroacetic acid (TCA). The reaction mixture is filtered through a cellulose acetate membrane (pore size: 0.45 µm) (Toyo Roshi) and after washing with 5% TCA four times, the radioactivity remaining on the membrane is determined. A control experiment is carried out without addition of the test compound and the radioactivity is determined in the same manner. The concentration of the test compound showing a 50% inhibition relative to the control is designated as $IC_{50}$. The results are shown in Table 4.

(2) Protein kinase A inhibition assay:

In accordance with the method of Kuo et al. (Biochemistry, 64, 1349, 1969), 10 µl of a solution of the test compound is added to 250 µl of a solution containing 5 µmole Tris-HCl buffer (pH 6.8), 2.5 µmole magnesium acetate, 100 µg of histone type IIS (Sigma),

0.25 nmole c-AMP and 200 μg of a crude enzyme (partially purified from the calf heart by the method of Kuo et al.). Then, the same procedure as the above-described protein kinase C inhibition assay is carried out to calculate $IC_{50}$. The results are shown in Table 4.

Table 4

| Compound No. | $IC_{50}$ (μg/ml) Protein kinase C | Protein kinase A | A/C* |
|---|---|---|---|
| 3 | 0.06 | 10 | 166.7 |
| 6 | 0.003 | 0.03 | 10.0 |
| 11 | 0.0031 | 0.16 | 51.6 |
| 12 | 0.012 | 0.15 | 12.5 |
| 13 | 0.002 | 0.036 | 18.0 |
| 15 | 0.0018 | 0.013 | 7.2 |
| 16 | 0.014 | 0.19 | 13.6 |
| 17 | 0.00068 | 0.12 | 176.5 |
| Staurosporine (Reference compound) | 0.001 | 0.004 | 4.0 |
| UCN-01 (Reference compound) | 0.002 | 0.02 | 10.0 |

* Activity ratio of protein kinase A to protein kinase C.

It will be apparent from Table 4 that compound (I) of this invention has selective protein kinase C-inhibitory activity as compared with reference compounds, suggesting, therefore, that reduced toxicity can be expected in its use as an antitumor agent.

What is claimed is:

1. A staurosporine derivative of the formula

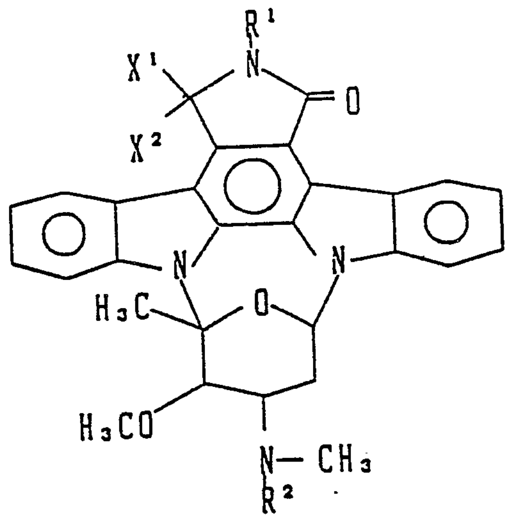

wherein $R^1$ represents hydrogen, lower alkyl, formyl or amino; $R^2$ represents hydrogen, formyl, lower alkanoyl or a group available upon elimination of the hydroxyl moiety of the carboxyl group of an α-amino acid, where the amino group of said amino acid may be protected by a protective group; one of $X^1$ and $X^2$ is hydrogen and the other is hydrogen, hydroxyl, lower alkoxyl or lower alkylthio or both of them, taken together, represent oxygen; provided, however, that where both $R^1$ and $R^2$ concurrently represent hydrogen and either $X^1$ or $X^2$ is hydrogen, the other is a group other than hydrogen and hydroxyl, or a pharmaceutically acceptable salt thereof.

# INTERNATIONAL SEARCH REPORT

International Application No PCT/JP89/00086

**I. CLASSIFICATION OF SUBJECT MATTER** (if several classification symbols apply, indicate all) [6]

According to International Patent Classification (IPC) or to both National Classification and IPC

Int. $Cl^4$ C07D498/22

**II. FIELDS SEARCHED**

Minimum Documentation Searched [7]

| Classification System | Classification Symbols |
|---|---|
| IPC | C07D498/22 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 60-185719 (Ajinomoto Co., Inc.)<br>21 September 1985 (21. 09. 85)<br>Claim (Family: none) | 1 |
| A | JP, A, 62-220196 (Kyowa Hakko Kogyo Co., Ltd.)<br>10 July 1987 (10. 07. 87)<br>Claim & EP, A, 238011 | 1 |
| P | EP, A, 296110 (CIBA-Geigy A.G.)<br>21 December 1988 (21. 12. 88)<br>& JP, A, 64-34989 | 1 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| March 24, 1989 (24. 03. 89) | April 17, 1989 (17. 04. 89) |
| **International Searching Authority** | **Signature of Authorized Officer** |
| Japanese Patent Office | |